# EUROPÄISCHE PATENTSCHRIFT

(11) **EP 1 074 215 B1**
(45) Veröffentlichungstag und Bekanntmachung des Hinweises auf die Patenterteilung: **13.08.2003**
(21) Anmeldenummer: 00110681.4
(22) Anmeldetag: 19.05.2000
(51) Int. Cl.: A61B 3/135, A61B 3/117

(54) **Spaltprojektor**
Slit projector
Projecteur à fente

(30) Priorität: 04.08.1999 DE 29913603 U
(43) Veröffentlichungstag der Anmeldung: 07.02.2001
(73) Patentinhaber: Oculus Optikgeräte GmbH, 35582 Wetzlar (DE)
(72) Erfinder: Köst, Gert, 30171 Hannover (DE); Repnow, Marc, 28213 Bremen (DE)
(74) Vertreter: Missling, Arne, Dipl.-Ing.

(56) Entgegenhaltungen:
- WO-A-00/33729
- WO-A-99/12467
- US-A- 4 699 482
- SASAKI K ET AL: "THE MULTI-PURPOSE CAMERA: A NEW ANTERIOR EYE SEGMENT ANALYSIS SYSTEM" OPHTHALMIC RESEARCH,CH,BASEL, Bd. 22, Nr. SUPPL. 01, 1. Mai 1990 (1990-05-01), Seiten 3-8, XP000613874

## Beschreibung

Die Erfindung betrifft einen Spaltprojektor mit einer Lichtquelle gemäß Oberbegriff der Ansprüche 1 und 3.

Ein derartiger Spaltprojektor ist aus dem Stand der Technik z.B. aus dem Buch "Ophtalmologisch optische Instrumente", herausgegeben von Bernhard Rassow, bekannt. Das Prinzip des Spaltprojektors, wie er z.B. in einer Spaltlampe verwendet wird, beruht darauf, dass die brechenden Medien der Augenvorderkammer nicht glasklar sind, sondern an ihnen insbesondere im kurzwelligen Anteil des sichtbaren Lichtes, eine deutliche Streuung erfolgt. Dies führt dazu, dass ein scharf gebündelter Lichtstrahl, dem man durch die optischen Medien des Auges schickt, bei seitlicher Betrachtung in ihnen sichtbar wird, ähnlich wie Lichtstrahlen eines Scheinwerfers im Nebel. Die verschiedenen Anteile der brechenden Medien des Auges haben eine unterschiedlich starke Lichtstreuung und können daher unterschieden werden.

Dieses Prinzip der fokalen Beleuchtung wird in dem Spaltprojektor perfektioniert. Zur Beleuchtung wird ein spaltförmiges Lichtbündel hoher Lichthelligkeit und möglichst hoher Farbtemperatur (kurzwelliger Bereich) verwendet.

Als Lichtquelle eines solchen Spaltprojektors hat sich in der Vergangenheit eine Niedervoltlampe durchgesetzt, weil sie eine hohe Leuchtdichte mit relativ hoher Farbtemperatur ermöglicht. Halogenlampen haben den Vorteil, dass sie im Dauerbetrieb höher belastet werden können und damit deutlich die Farbtemperatur steigt, außerdem wird ihr Licht durch Halterung nur unwesentlich verändert. Nur in einer Sonderausführung einer extrem aufwendig gebauten Spaltlampe gibt es als Lichtquelle eine Xenon-Hochdrucklampe.

Von Augenärzten werden Spaltprojektoren eingesetzt, mit denen neben der Beobachtung der Augenvorderkammer auch Fotografien des Schnittbildes der Augenvorderkammer angefertigt werden können. Gute Aufnahmen des vorderen Augenabschnittes setzten dabei eine ziemlich starke Abblendung des verwendeten Aufnahmeobjektivs voraus, um eine gute Schärfentiefe zu erzielen. Das bedeutet, dass die Lichtquellen in der Beleuchtung sehr leistungsfähig sein müssen. Fotografien des vorderen Augenabschnittes im optischen Schnitt sind bisher nur mit in die Spaltbeleuchtung eingekuppeltem Elektronenblitz bzw. der Xenon-Hochdrucklampe möglich. Bei dem Spaltprojektor mit Xenon-Hochdrucklampe handelt es sich um ein hochgezüchtetes und sehr teures Spitzengerät, das für die gesamte Diagnostik ebenso wie für die Fotografie hervorragend einsetzbar ist. Dieser Spaltprojektor übertrifft aber in seiner Ausstattung und auch im Preis bei weitem für die übliche Diagnostik zu stellenden Anforderungen.

Ein weiterer Nachteil bei der Verwendung von herkömmlichen Elektronenblitzen für die Schnittbild-Fotografie ist die lange Aufladdauer des Elektronenblitzes. Zwischen den Fotografien muss daher immer mehrere Sekunden gewartet werden, bis der Elektronenblitz erneut bereit ist und eine weitere Aufnahme gemacht werden kann. Eine gesamte Erfassung der Augenvorderkammer mittels einer Fotospaltlampe ist daher zeitaufwendig und teuer.

Der Erfindung liegt daher die Aufgabe zugrunde, einen Spaltprojektor vorzuschlagen, der einen Spalt projiziert, der die nötige Helligkeit zum Beobachten des Auges und insbesondere auch zur Schnittbild-Fotografie bereitstellt und eine hohe Bildfrequenz ermöglicht.

Diese Aufgabe wird erfindungsgemäß durch die merkmalen der Anspruch 1 und 3 gelöst, wobei die Lichtquelle aus mehreren im Wesentlichen in Spaltlängsrichtung, d. h. in der Ebene des projizierten Spaltes angeordneten Leuchtdioden besteht.

Der erfindungsgemäße Einsatz von Leuchtdioden als Lichtquelle des Spaltprojektors bringt gleich mehrere Vorteile. Der für den Augenarzt bedeutendste Vorteil liegt in der hohen Geschwindigkeit, mit der die für die Schnittbild-Fotografie notwendigen Blitze mit den Leuchtdioden erzeugt werden können. Darüber hinaus sind Leuchtdioden wesentlich kompakter als die herkömmlichen Lichtquellen, die Leuchtdioden sind robuster als die hochempfindlichen Xenon-Hochdrucklampen und spürbar preiswerter.

Gemäß der Erfindung können die Leuchtdioden dann in einer ersten Ausführungsform in der Ebene des projizierten Spaltes liegend bogen- oder kreisförmig angeordnet sein. Die Krümmung des Bogen ist dann vorteilhaft so bestimmt, dass das Linsensystem von einer größtmöglichen Lichtmenge passiert wird.

In einer anderen Ausführungsform können die Leuchtdioden erfindungsgemäß im Wesentlichen in einer Ebene parallel zu der Spaltblende angeordnet sein. Erfindungsgemäß sind dann Hauptstrahlen der Leuchtdioden relativ zur optischen Achse des Linsensystems geneigt und die Neigung der Hauptstrahlen ist dem Abstand der Leuchtdioden zur optischen Achse des Linsensystems proportional. Vorteilhaft schneiden sich dann die Hauptstrahlen aller Leuchtdioden im Wesentlichen in einem gemeinsamen Punkt.

Ein Spaltprojektor kann vorteilhaft vor dem Linsensystem eine zweite Spaltblende aufweisen, wobei der Spalt dieser zweiten Spaltblende mit dem Spalt der ersten Spaltblende fluchtet.

Vorzugsweise können die Leuchtfelder aus Leuchtdioden-Chips bestehen. Diese Leuchtdioden-Chips sind dann vorteilhaft entlang einer Geraden angeordnet, wobei die Anschlussfelder beiderseits der Geraden liegen. Durch diese Anordnung wird erreicht, dass die durch die Anschlussfelder abgeschatteten Bereiche der Leuchtdioden-Chips, möglichst am Rande der auf die erste Spaltblende abgebildeten Leuchtfelder liegen und eine größtmögliche Lichtmenge das Linsensystem erreicht.

Bei besonderen Ausführungsformen können die Diodenlinsen astigmatisch sein und/oder das Linsensystem aus Zylinderlinsen bestehen. In beiden Fällen wird eine Bündelung des von den Leuchtfeldern erzeugten Lichtes auf den Spalt in der ersten Spaltblende erreicht.

Diese erste Spaltblende kann Vorzugsweise eine Breite von 50 bis 120 µm haben, während der Öffnungswinkel des aus dieser ersten Spaltblende austretenden Strahlenbündels kleiner gleich 2,9° sein soll.

Um ein vorteilhaftes Streuverhalten im Auge zu erhalten, werden Leuchtdioden bevorzugt, welche blaues Licht erzeugen.

Die Erfindung ist anhand der Zeichnung näher beschrieben. Darin zeigt
- Fig. 1: eine schematische Seitenansicht eines Spaltprojektors
- Fig. 2: die Draufsicht auf den Strahlenprojektor gemäß Fig. 1

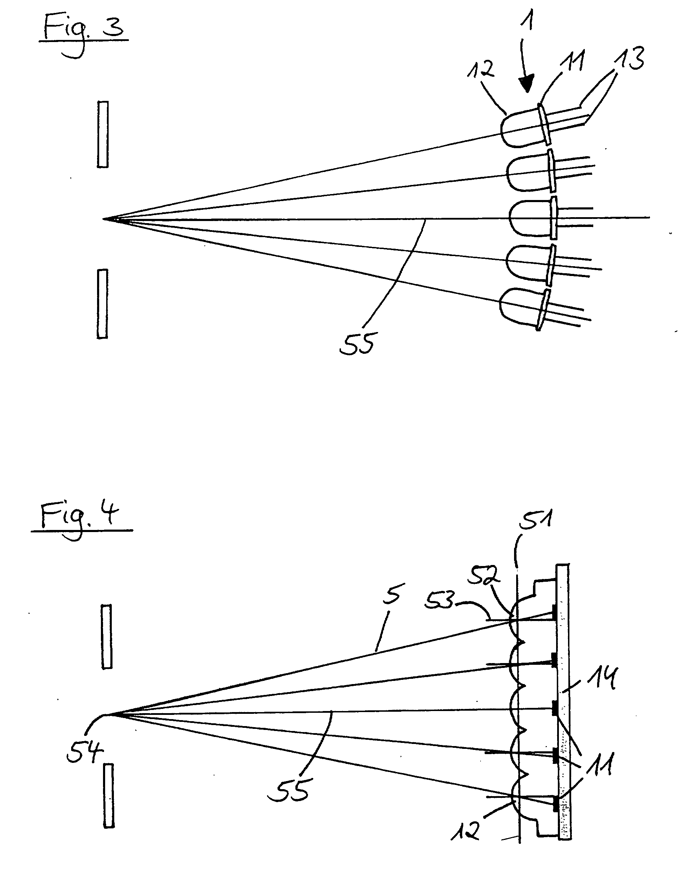

- Fig. 3: eine schematische Darstellung der Anordnung der Dioden einem Strahlenprojektor gemäß Fig. 1 und 2
und
- Fig. 4: eine schematische Darstellung der Anordnung der Leuchtdioden in einem anderen Strahlenprojektor.

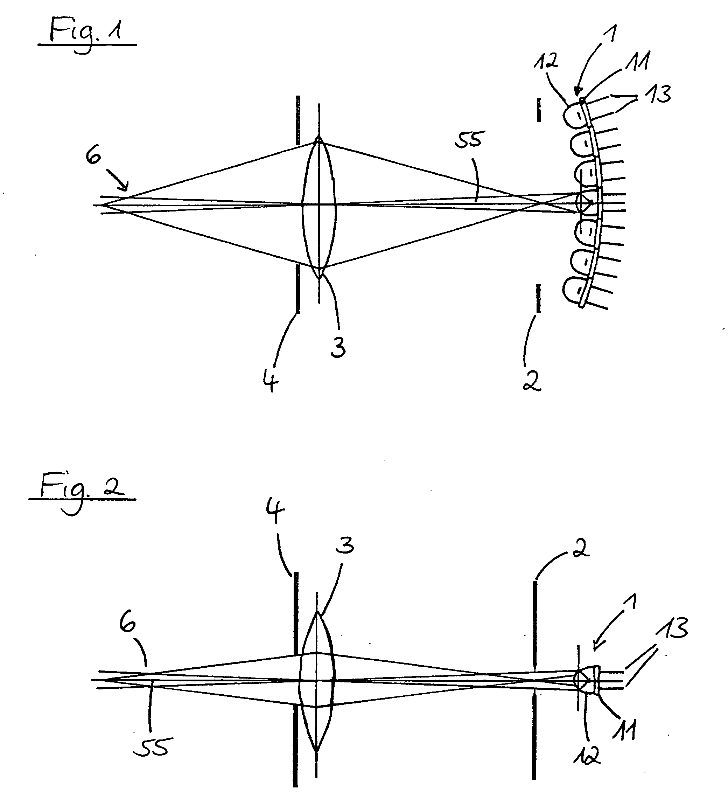

Bei dem in Fig. 1 und 2 schematisch dargestellten Ausführungsbeispiel eines Spaltprojektors sind Leuchtdioden 1 auf einem Kreisbogen angeordnet. Die Leuchtdioden 1 beleuchten eine erste Spaltblende 2 vor der eine Linse 3 angeordnet ist. Die Krümmung des Kreisbogens ist dabei so gewählt, dass eine möglichst große Lichtmenge durch die Linse 3 hindurchtritt. Vor der Linse 3 ist eine weitere zweite Spaltblende 4 angeordnet, welche zu einer weiteren Bündelung des Lichtstrahls führt.

In Fig. 1 ist der Strahlengang der Leuchtdiode eingezeichnet, dessen Hauptstrahl mit der optischen Achse 55 des Spaltprojektors zusammenfällt. In Fig. 2 ist der gleiche Strahlengang in einem horizontalen Querschnitt dargestellt. Die Abbildung der Leuchtfelder der Leuchtdiode 1 erfolgt dabei nach dem aus dem Stand der Technik bekannten Köhlerschen Beleuchtungsprinzip. Dabei wird das Leuchtfeld der Leuchtdiode 1 in die Linse 3 abgebildet. Diese wiederum bildet die erste Spaltblende, welche direkt vor der Leuchtdiode 1 liegt und gleichmäßig beleuchtet ist, in das Auge eines Probanden ab. Sowohl in Fig. 1 als auch in Fig. 2 ist ein Bereich starker Bündelung zu erkennen, in welchem das von der Leuchtdiode 1 erzeugte Licht möglichst stark gebündelt ist. In diesem Bereich 6 befindet sich bei den Augenuntersuchungen vorteilhaft das Auge des Probanden.

Die Leuchtdioden 1 sind auf einem Kreisbogen angeordnet, so dass sich die Hauptstrahlen in einem gemeinsamen Punkt schneiden. Dadurch ist gewährleistet, dass eine möglichst große Lichtmenge durch die Linse 3 hindurchtritt. Der gemeinsame Schnittpunkt der Hauptstrahlen liegt dabei vorteilhaft in der zweiten Spaltblende 4, in der Linse 3 oder zwischen der zweiten Spaltblende 4 und der Linse 3.

Eine andere erfindungsgemäße Anordnung (Fig. 4) der Leuchtdioden 1 führt ebenfalls zu einer starken Bündelung des Lichtes wie bei dem vorbeschriebenen Ausführungsbeispiel. Bei dieser Anordnung der Leuchtdioden 1 sind die Leuchtfelder 11 entlang einer Geraden auf einer Trägerplatine 14 angebracht. Vor den Leuchtfeldern sind die Diodenlinsen 12 ebenfalls auf einer Geraden angebracht. Dabei sind die Leuchtfelder 11 der Leuchtdiode 1 in gleicher Richtung relativ zu der Diodenlinse 12 verschoben, wie die Diodenlinse 12 zur optischen Achse 55 des Linsensystems bzw. des Spaltprojektors verschoben ist. Die Verschiebung des Leuchtfeldes 11 ist dabei proportional zu dem Abstand der Diodenlinsen 12 zur optischen Achse 55. Die Hauptstrahlen 5 aller Leuchtdioden 1 schneiden sich dabei in einem gemeinsamen Punkt 54. Dabei sind die Hauptstrahlen 5 die Strahlen, welche vom Mittelpunkt eines Leuchtfeldes 11 ausgehend, durch den Schnittpunkt der Hauptebene 51 der Diodenlinse 12 mit der optischen Achse der Diodenlinse 53 hindurchtreten. Durch diese Anordnung der Diodenlinsen 12 und Leuchtfelder 11 zueinander wird erreicht, dass die Leuchtfelder 11 in der Linse 3 des Spaltprojektors abgebildet werden. So wird entsprechend dem Köhlerschen Beleuchtungsprinzip eine größtmögliche Bündelung der Strahlen bzw. eine größtmögliche Helligkeit des beleuchteten Spaltes erreicht.

Im Gegensatz zu den herkömmlichen und aus dem Stand der Technik bekannten Lichtquellen, haben die Leuchtdioden den Vorteil, dass sie wesentlich robuster und einfach zu handhaben sind. Mit Leuchtdioden kann ein Spaltprojektor für wesentlich längere Zeit betrieben werden, als beispielsweise mit Xenon-Hochdrucklampen, ohne dass es zu Ausfällen kommt. Vorteilhaft ist bei den Leuchtdioden insbesondere, dass bei Leuchtdioden eine wesentlich geringere Wärmeentwicklung auftritt, so dass eine besondere Kühlung der Lichtquelle nicht vorgesehen werden muß. Des Weiteren eignen sich Spaltprojektoren mit Leuchtdioden besonders gut zur Schnittbild-Fotografie, da eine Aufladung von Kondensatoren zum Bereitstellen der Blitzspannung nicht notwendig ist. Bei den Leuchtdioden muss lediglich für den Zeitpunkt der Fotografie die Diodenspannung beispielsweise auf den fünffachen Wert über den Sättigungswert der Rezeptoren des Auges erhöht werden. Zwischen den einzelnen Fotografien liegen beispielsweise 80 bis 100 ms, so dass die Blitzfolge gegenüber einer Xenon-Hochdruckblitzlampe um ein Vielfaches gesteigert ist.

### Bezugszeichenliste

- 1: - Leuchtdiode
- 11: - Leuchtfeld
- 12: - Diodenlinse
- 13: - Anschlussleitungen
- 14: - Trägerplatine

- 2: - erste Spaltblende
- 3: - Linse
- 4: - zweite Spaltblende
- 5: - Hauptstrahl
- 51: - Hauptebene
- 52: - Hauptpunkt
- 53: - optische Achse einer Diodenlinse
- 54: - gemeinsamer Schnittpunkt
- 55: - optische Achse des Spaltprojektors

- 6: - Bereich starker Bündelung

## Patentansprüche

1. Spaltprojektor, mit einer Lichtquelle, einer vor der Lichtquelle angeordneten Spaltblende, und einem der Spaltblende nachgeordnetem Linsensystem, insbesondere für Spaltlampen,
**dadurch gekennzeichnet,**
**dass** die Lichtquelle aus mehreren im Wesentlichen in Spaltlängsrichtung, d. h. in der Ebene des projizierten Spaltes angeordneten Leuchtdioden (1) besteht, wobei die
Leuchtdioden (1) in der Ebene des projizierten Spaltes liegend bogen- oder kreisförmig angeordnet sind.

2. Spaltprojektor nach Anspruch 1, **dadurch gekennzeichnet, dass** die Krümmung des Bogens so bestimmt ist, dass das Linsensystem (3) von einer größtmöglichen Lichtmenge passiert wird.

3. Spaltprojektor, mit einer Lichtquelle, einer vor der Lichtquelle angeordneten Spaltblende, und einem der Spaltblende nachgeordnetem Linsensystem, insbesondere für Spaltlampen,
**dadurch gekennzeichnet,**
**dass** die Lichtquelle aus mehreren im Wesentlichen in Spaltlängsrichtung, d. h. in der Ebene des projizierten Spaltes angeordneten Leuchtdioden (1) besteht, wobei die
Leuchtdioden (1) im Wesentlichen in einer Ebene parallel zu der Spaltblende (2) angeordnet sind, und wobei die
Hauptstrahlen (5) der Leuchtdioden (1) relativ zur optischen Achse des Linsensystems (3) geneigt sind und dass die Neigung der Hauptstrahlen (5) dem Abstand der Leuchdioden (1) zur optischen Achse des Linsensystems (3) proportional ist.

4. Spaltprojektor nach Anspruch 3, **dadurch gekennzeichnet, dass** die Hauptstrahlen aller Leuchtdioden (1) sich im Wesentlichen in einem gemeinsamen Punkt schneiden.

5. Spaltprojektor, nach einem der Ansprüche 1 bis 4, **dadurch gekennzeichnet, dass** vor dem Linsensystem (3) eine zweite Spaltblende (4) angeordnet ist, wobei der Spalt dieser zweiten Spaltblende (4) mit dem Spalt der ersten Spaltblende (2) gleichachsig ist und fluchtet.

6. Spaltprojektor nach einem der Ansprüche 1 bis 5, **dadurch gekennzeichnet, dass** die Leuchtfelder (11) der Leuchtdioden (1) aus Leuchtdioden-Chips bestehen.

7. Spaltprojektor nach Anspruch 6, **dadurch gekennzeichnet, dass** die Leuchtdioden-Chips (11) entlang einer Geraden angeordnet sind, wobei deren Anschlussfelder beiderseits der Geraden liegen.

8. Spaltprojektor nach einem der Ansprüche 1 bis 7, **dadurch gekennzeichnet, dass** die Diodenlinse (12) astigmatisch sind.

9. Spaltprojektor nach einem der Ansprüche 1 bis 9, dadurch gekenn-5 zeichnet, dass das Linsensystem aus Zylinderlinsen (3) besteht.

10. Spaltprojektor nach einem der Ansprüche 1 bis 9, **dadurch gekennzeichnet, dass** der Spalt der ersten Spaltblende (2) eine Breite von 50 bis 120 µm hat.

11. Spaltprojektor nach einem der Ansprüche 1 bis 10, **dadurch gekennzeichnet, dass** der Öffnungswinkel des aus der ersten Spaltblende (2) austretenden Strahlenbündels kleiner gleich 2,9° ist.

12. Spaltprojektor nach einem der Ansprüche 1 bis 11, **dadurch gekennzeichnet, dass** die Leuchtdioden (1) blaues Licht erzeugen.

## Claims

1. A slit projector, having a light source, a slit diaphragm arranged in front of the light source, and a lens system arranged downstream of the slit diaphragm, in particular for slit lamps,
**characterised in that**
the light source comprises a plurality of light emitting diodes (1) arranged substantially in the longitudinal direction of the slit, that is to say in the plane of the projected slit, with the light emitting diodes (1) being arranged in the shape of an arc or a circle lying in the plane of the projected slit.

2. A slit projector according to Claim 1, **characterised in that** the curvature of the arc is determined such that the greatest possible quantity of light passes through the lens system (3).

3. A slit projector, having a light source, a slit diaphragm arranged in front of the light source, and a lens system arranged downstream of the slit diaphragm, in particular for slit lamps,
**characterised in that**
the light source comprises a plurality of light emitting diodes (1) arranged substantially in the longitudinal direction of the slit, that is to say in the plane of the projected slit, with the light emitting diodes (1) being arranged substantially in a plane parallel to the slit diaphragm (2), and with the principal rays (5) of the light emitting diodes (1) being inclined relative to the optical axis of the lens system (3), and **in that** the inclination of the principal rays (5) is proportional to the spacing between the light emitting diodes (1) and the optical axis of the lens system (3).

4. A slit projector according to Claim 3, **characterised in that** the principal rays of all the light emitting diodes (1) intersect one another substantially at a common point.

5. A slit projector according to one of Claims 1 to 4, **characterised in that** a second slit diaphragm (4) is arranged in front of the lens system (3), with the slit of this second slit diaphragm (4) coaxial with the slit of the first slit diaphragm (2) and in alignment therewith.

6. A slit projector according to one of Claims 1 to 5, **characterised in that** the illuminated fields (11) of the light emitting diodes (1) comprise light emitting diode chips.

7. A slit projector according to Claim 6, **characterised in that** the light emitting diode chips (11) are arranged along a straight line, with the terminal panels thereof lying on either side of the straight line.

8. A slit projector according to one of Claims 1 to 7, **characterised in that** the diode lenses (12) are astigmatic.

9. A slit projector according to one of Claims 1 to 9, **characterised in that** the lens system comprises cylindrical lenses (3).

10. A slit projector according to one of Claims 1 to 9, **characterised in that** the slit of the first slit diaphragm (2) has a width of 50 to 120 µm.

11. A slit projector according to one of Claims 1 to 10, **characterised in that** the aperture angle of the bundle of rays emerging from the first slit diaphragm (2) is less than or equal to 2.9°.

12. A slit projector according to one of Claims 1 to 11, **characterised in that** the light emitting diodes (1) generate blue light.

## Revendications

1. Projecteur à fente, avec une source lumineuse, un diaphragme à fente placé devant la source lumineuse, et un système de lentilles placé en aval du diaphragme à fente, en particulier pour lampes à fente,
**caractérisé en ce que**
la source lumineuse est composée de plusieurs diodes électroluminescentes (1) disposées essentiellement dans le sens longitudinal de la fente, c'est-à-dire dans le plan de la fente projetée,
les diodes électroluminescentes (1) étant disposées à plat en forme de cercle ou d'arc dans le plan de la fente projetée.

2. Projecteur à fente selon la revendication 1, **caractérisé en ce que** la courbe de l'arc est déterminée de telle sorte que le système de lentilles (3) est traversé par une quantité lumineuse maximale.

3. Projecteur à fente, avec une source lumineuse, un diaphragme à fente placé devant la source lumineuse, et un système de lentilles placé en aval du diaphragme à fente, en particulier pour lampes à fente,
**caractérisé en ce que**
la source lumineuse est composée de plusieurs diodes électroluminescentes (1) disposées essentiellement dans le sens longitudinal de la fente, c'est-à-dire dans le plan de la fente projetée,
les diodes électroluminescentes (1) étant disposées essentiellement dans un plan parallèle au diaphragme à fente (2), et
les rayons principaux (5) des diodes électroluminescentes (1) étant inclinés par rapport à l'axe optique du système de lentilles (3), et l'inclinaison des rayons principaux (5) étant proportionnelle à la distance entre les diodes électroluminescentes (1) et l'axe optique du système de lentilles (3).

4. Projecteur à fente selon la revendication 3, **caractérisé en ce que** les rayons principaux de toutes les diodes électroluminescentes (1) se coupent essentiellement en un point commun.

5. Projecteur à fente, selon l'une quelconque des revendications 1 à 4, **caractérisé en ce qu'**un second diaphragme à fente (4) est placé devant le système de lentilles (3), la fente de ce second diaphragme à fente (4) étant alignée sur, et sur le même axe que, la fente du premier diaphragme à fente (2).

6. Projecteur à fente selon l'une quelconque des revendications 1 à 5, **caractérisé en ce que** les champs lumineux (11) des diodes électroluminescentes (1) sont composés de puces à diodes électroluminescentes.

7. Projecteur à fente selon la revendication 6, **caractérisé en ce que** les puces à diodes électroluminescentes (11) sont disposées le long d'une ligne droite, les champs de raccordement de celles-ci se trouvant des deux côtés de la ligne droite.

8. Projecteur à fente selon l'une quelconque des revendications 1 à 7, **caractérisé en ce que** les lentilles de diodes (12) sont astigmates.

9. Projecteur à fente selon l'une quelconque des revendications 1 à 8, **caractérisé en ce que** le système de lentilles est composé de lentilles cylindriques (3).

10. Projecteur à fente selon l'une quelconque des revendications 1 à 9, **caractérisé en ce que** la fente du premier diaphragme à fente (2) a une largeur comprise entre 50 et 120 µm.

11. Projecteur à fente selon l'une quelconque des revendications 1 à 10, **caractérisé en ce que** l'angle de divergence du faisceau lumineux qui émerge du premier diaphragme à fente (2) est légèrement inférieur à 2,9°.

12. Projecteur à fente selon l'une quelconque des revendications 1 à 11, **caractérisé en ce que** les diodes électroluminescentes (1) produisent de la lumière bleue.
